# EUROPEAN PATENT APPLICATION

(11) **EP 4 282 436 A1**
(43) Date of publication of application: **29.11.2023**
(21) Application number: 22742499.1
(22) Date of filing: 14.01.2022
(51) Int. Cl.: A61K 49/04, A61B 6/03, A61K 47/64

(54) **COMPLEX, VASCULAR CONTRAST AGENT, X-RAY CONTRAST AGENT, METHOD FOR PRODUCING COMPLEX, AND IMAGING METHOD FOR CAPTURING STRUCTURAL CHANGE IN VESSEL**

(30) Priority: 22.01.2021 JP 2021008669
(71) Applicant: Tohoku University, Sendai-shi, Miyagi 980-8577 (JP)
(72) Inventor: INOSE Tomoya, Sendai-shi, Miyagi 980-8577 (JP); TAKASE Takumu, Sendai-shi, Miyagi 980-8577 (JP); KITAMURA Narufumi, Sendai-shi, Miyagi 980-8577 (JP); GONDA Kohsuke, Sendai-shi, Miyagi 980-8577 (JP)
(74) Representative: Heyerhoff Geiger GmbH & Co. KG
(86) International application number: PCT/JP2022/001033
(87) International publication number: WO 2022/158377

(57) **Abstract**

The present invention addresses the problem of providing an x-ray contrast agent that resides for a predetermined time in a blood vessel and is then excreted outside the body.

The problem can be solved by a complex including metal nanoparticles and a protein, wherein the metal nanoparticle portion of the complex has an average particle size of 1-5.5 nm (inclusive).

## Description

### Technical Field

The present disclosure relates to a complex, a vascular contrast agent, an X-ray contrast agent, a method for producing a complex, and an imaging method for capturing structural change in a vessel.

### Background Art

X-ray computed tomography (CT) imaging is an extremely useful measurement method that enables in vivo, three-dimensional, high-resolution visualization of a lesion site of cancer and the like in a living body. Hard tissues, such as bones and teeth, absorb X-rays well, and thus high-contrast images can be obtained. However, soft tissues, such as cancer cells and ordinary cells, have a small difference in X-ray absorption, and thus high contrast cannot be obtained. Thus, a contrast agent is usually used to obtain high-contrast images in soft tissues.

For X-ray contrast agents, compounds with a water-solubilized triiodophenyl group have been used in the art. In addition to a triiodophenyl group, a metal fine particle complex composed of a zero-valent transition metal fine particle with an average particle size of 2.5 nm and a nonionic hydrophilic ligand for dispersing and stabilizing the fine particle in water is known to be used as an X-ray contrast agent (see Patent Document 1).

However, X-ray contrast agents prepared from a compound with a water-solubilized triiodophenyl group and the X-ray contrast agent described in Patent Document 1 are excreted from a luminal portion without interacting with a tissue or a disease site.

A known example of an X-ray contrast agent for solving the above problem includes a metal fine particle complex containing a compound containing a zero-valent transition metal fine particle and a polyalkylene glycol moiety reacted with transferrin, which is known to be able to maintain a contrast level particularly in a tumor portion when used as an X-ray contrast agent (see Patent Document 2).

In addition, another X-ray contrast agent is also known, which is obtained by binding an X-ray absorbing nanoparticle and a fluorescent nanoparticle and thus functions as an X-ray CT contrast agent for specifically imaging a focus of cancer and the like and also functions as a fluorescent labeling agent (see Patent Document 3). Patent Document 3 also describes that the above X-ray contrast agent enables dual-mode imaging with X-rays observable from outside the body using X-ray CT or the like and highly sensitive fluorescence, and thus enables real-time identification of the location, size, and range of a focus without using expensive positron emission tomography (PET) imaging when treatment, such as surgery, is performed, allowing inexpensive diagnosis and treatment.

### Citation List

### Patent Literature

Patent Document 1: JP H10-330288 A
Patent Document 2: JP 2005-120034 A
Patent Document 3: WO 2012/153820

### Summary of Invention

### Technical Problem

Tumor blood vessels play an important role in the growth and metastasis of cancer tissues. Thus, a technique for accurately visualizing the structure of tumor blood vessels is extremely important in elucidating the mechanism of cancer and evaluating the efficacy of anticancer agents. X-ray CT imaging of blood vessels is performed by administering a contrast agent usually by intravenous infusion and performing imaging at the optimum timing. Meanwhile, the blood vessels of experimental animals, such as mice, are very thin, and thus a micro X-ray CT device is often used for imaging blood vessels. This micro X-ray CT device requires imaging time in exchange for high resolution. However, X-ray contrast agents in the art prepared from a compound with a water-solubilized triiodophenyl group and the X-ray contrast agent described in Patent Document 1 have a short residence time in the blood and thus are accompanied by a problem of difficulty in obtaining X-ray CT images of blood vessels.

On the other hand, the X-ray contrast agent described in Patent Document 3 can obtain X-ray CT images of tumor tissues. However, the X-ray contrast agent is not excreted out of the body and remains in tumor tissues after extravasation, thus this makes it difficult to obtain contrast images of X-rays derived from tumor blood vessels and results in a problem of difficulty in visualizing structural change in tumor blood vessels over time, which has been newly found. At present, however, no X-ray contrast agent is known, which is retained in the blood vessels for a predetermined period of time and then excreted out of the body.

The disclosure in the present application has been made to solve the problems described above. A study has been diligently conducted and has newly found that forming a complex using a protein enables even a metal nanoparticle with a size that would otherwise allow renal excretion to be used as a material for a contrast agent suitable for X-ray CT imaging of vessels, such as blood vessels.

That is, an object of the disclosure in the present application is to provide a complex that can be used as a material for a contrast agent for X-ray CT imaging of a vessel, a vascular contrast agent and an x-ray contrast agent containing the complex, a method for producing a complex, and an imaging method for capturing structural change in a vessel.

### Solution to Problem

(1) A complex containing a metal nanoparticle and a protein, wherein
   a metal nanoparticle part in the complex has an average particle size of 1 nm or greater and 5.5 nm or less.
(2) The complex according to (1), wherein
   the metal nanoparticle and the protein are linked via a linker.
(3) The complex according to (2), wherein
   the linker before forming the complex is a compound containing a thiol group and a carboxyl or amino group, and
   in the complex,
   a S atom of the thiol group is bound to a metal in the metal nanoparticle, and
   the carboxyl group or the amino group is bound via an amide bond to an amino group or a carboxyl group of the protein.
(4) The complex according to any one of (1) to (3), wherein
   the protein is selected from functional proteins.
(5) The complex according to any one of (1) to (4), wherein
   the complex has a half-life in blood from 1 to 3 hours.
(6) The complex according to any one of (1) to (5), wherein
   in the complex, the protein functions as a core, and the metal nanoparticle is bound to a surface of a protein core provided by the protein.
(7) The complex according to any one of (1) to (6), wherein
   a metal of the metal nanoparticle is selected from the group consisting of gold, platinum, and alloys of gold and platinum.
(8) A vascular contrast agent containing the complex described in any one of (1) to (7).
(9) An X-ray contrast agent containing the complex described in any one of (1) to (7).
(10) A method for producing a complex using a metal nanoparticle, a protein, and a linker compound, a metal nanoparticle part in the complex having an average particle size of 1 nm or greater and 5.5 nm or less, the linker compound having a thiol group and a carboxyl or amino group, and the method including:
   preparing a linker-bound metal nanoparticle by reacting the metal nanoparticle with the linker compound to bind a S atom of the thiol group to a metal in the metal nanoparticle; and
   binding the carboxyl group or the amino group of the linker-bound metal nanoparticle to an amino group or a carboxyl group of the protein via an amide bond by reacting the linker-bound metal nanoparticle with the protein.
(11) An imaging method for capturing structural change in a vessel using a complex, the complex having a metal nanoparticle and a protein, a metal nanoparticle part in the complex having an average particle size of 1 nm or greater and 5.5 nm or less, and
   the imaging method including:
   administering the complex to an animal and performing X-ray CT imaging, and after an appropriate time has elapsed, administering a new complex and performing X-ray CT imaging.

### Advantageous Effects of Invention

When the complex disclosed in the present application is used as a contrast agent, structural change in a vessel over time can be visualized.

### Brief Description of Drawings

FIG. 1 is a set of photographs substituted for drawings. FIG. 1A is a transmission electron microscope (TEM) photograph of a sAu/GSH-LF prepared in Example 1. FIG. 1B is a TEM photograph of a sAu/GSH prepared in Comparative Example 1. FIG. 1C is a TEM photograph of a AuNP-PEG prepared in Comparative Example 2.
FIG. 2 is a photograph substituted for a drawing and a photograph after electrophoreses of a sAu/GSH-LF colloidal solution prepared in Example 1 and a sAu/GSH colloidal solution prepared in Comparative Example 1.
FIG. 3 is a graph showing ultraviolet-visible (UV-VIS) spectral measurement results of the sAu/GSH-LF colloidal solution prepared in Example 1 and the sAu/GSH colloidal solution prepared in Comparative Example 1.
FIG. 4 is a set of photographs substituted for drawings. FIGS. 4a to 4d are micro X-ray CT images after administration of a contrast agent prepared in Comparative Example 1 to the tail vein of a mouse.
FIG. 5 is a set of photographs substituted for drawings. FIGS. 5A and 5B are micro X-ray CT images after administration of a contrast agent prepared in Comparative Example 2 to the tail vein of a mouse.
FIG. 6 is a set of photographs substituted for drawings showing CT images of mouse hearts before and after intravenous administrations of a sAu/GSH-LF contrast agent prepared in Example 1, a sAu/GSH contrast agent prepared in Comparative Example 1, and iopamidol of Comparative Example 3.
FIG. 7 is a graph showing results of measuring changes in CT values of ventricles of mouse hearts over time before and after intravenous administrations of the sAu/GSH-LF contrast agent prepared in Example 1, the sAu/GSH contrast agent prepared in Comparative Example 1, and iopamidol of Comparative Example 3.
FIG. 8a is a diagram illustrating an outline of an experiment to visualize changes in the same blood vessels associated with tumor growth using the sAu/GSH-LF contrast agent prepared in Example 1, the sAu/GSH contrast agent prepared in Comparative Example 1, and a 15-nm AuNP-PEG contrast agent prepared in Comparative Example 2. FIGS. 8b to 8j are photographs substituted for drawings, showing CT images of tumor portions.
FIG. 9 is a set of photographs substituted for drawings. FIG. 9a is an enlarged image of FIG. 8i, and FIG. 9b is an enlarged image of FIG. 8j.
FIG. 10 is photographs substituted for drawings and TEM photographs of sAu/GSH-hSA 1 prepared in Example 2.
FIG. 11 is a photograph substituted for a drawing and a photograph after electrophoreses of the colloidal solution (sAu/GSH-hSA 1) prepared in Example 2, the colloidal solution (sAu/GSH-hSA 2) prepared in Example 3, and the colloidal solution (sAu/GSH) prepared in Comparative Example 1.
FIG. 12 is a graph showing UV-VIS spectral measurement results of the colloidal solution prepared in Example 2, the colloidal solution prepared in Example 3, and the colloidal solution prepared in Comparative Example 1.
FIG. 13 is a graph showing measurement results of isoelectric points of the colloidal solution prepared in Example 2, the colloidal solution prepared in Example 3, and the colloidal solution prepared in Comparative Example 1.
FIG. 14 is a set of photographs substituted for drawings and micro X-ray CT images after administrations of the contrast agents prepared in Examples 2 and 3 and Comparative Example 1 to the tail veins of mice.
FIG. 15 is a set of photographs substituted for drawings and micro X-ray CT images (axial cross sections of the kidneys of mice) after administrations of the contrast agents prepared in Examples 2 and 3 and Comparative Example 1 to the tail veins of mice.
FIG. 16 is a set of photographs substituted for drawings and CT images (axial cross sections of the hearts of mice) before and after intravenous administrations of the contrast agents prepared in Examples 2 and 3 and Comparative Example 1.
FIG. 17 is a set of photographs substituted for drawings and CT images of mouse hearts (coronal cross sections of the hearts of mice) before and after intravenous administrations of the contrast agents prepared in Examples 2 and 3 and Comparative Example 1.
FIG. 18 is a graph showing results of measuring changes in CT values of ventricles of mouse hearts over time before and after intravenous administrations of the contrast agents prepared in Examples 2 and 3 and Comparative Example 1.
FIG. 19 is a photograph substituted for a drawing and a photograph of a linker-bound gold nanoparticle solution prepared in Reference Example 1.

### Description of Embodiments

Hereinafter, a complex, a vascular contrast agent, an x-ray contrast agent, a method for producing a complex, and an imaging method for capturing structural change in a vessel disclosed in the present application will be described in detail.

In the present specification, a numerical range represented using "to" means a range including numerical values described before and after "to" as a lower limit value and an upper limit value. In addition, in the present specification, numerical values, numerical ranges, and qualitative expressions (e.g., expressions, such as "identical" and "the same") are interpreted as indicating numerical values, numerical ranges, and properties including errors generally accepted in the art.

### Embodiment of Complex

An embodiment of a complex contains a metal nanoparticle and a protein.

### Metal Nanoparticle

The lower limit of the average particle size of the metal nanoparticle is preferably 1 nm or greater and may be 1.25 nm or greater, 1.5 nm or greater, 1.75 nm or greater, or 2 nm or greater. With the average particle size of less than 1 nm, the absorption amount of X-rays would be small, and this would make it difficult to obtain a high-contrast image. In addition, with the average particle size of less than 1 nm, the metal nanoparticle would have smaller specific surface area, and this would make it difficult to form a complex with the protein.

On the other hand, for the upper limit of the average particle size of the metal nanoparticle, the present inventors have confirmed from the results of comparative examples described later that the metal nanoparticle with a small average particle size is excreted by the kidneys, but the metal nanoparticle with a large average particle size remains in the tumor tissue. The metal nanoparticle with a large average particle size remains in the tumor tissue probably because of the enhanced permeation and retention effect (EPR effect). That is, the metal nanoparticle with a large average particle size is not excreted by the kidneys and thus is retained in the blood, and this probably resulted in exhibiting the EPR effect. Thus, to perform X-ray CT imaging of a blood vessel to enable visualization of structural change in a blood vessel over time, the metal nanoparticle is required to have a size that allows renal excretion from the body.

The glomerulus, an important tissue for renal excretion, is composed of blood vessels, the glomerular filtration membrane, and the Bowman's capsule. Among them, the glomerular membrane, important as a filtration function, has a multilayer structure composed of glycoproteins, endothelial cells, a glomerular basement membrane, and podocytes. The slit interval of the filtration function is from 70 to 90 nm for the endothelial cells, from 2 to 8 nm for the glomerular basement membrane, and from 4 to 11 nm for the podocytes. Thus, nanoparticles and proteins with a size less than 6 nm are known to be able to pass through the glomerular filtration membrane, but particles with a size of 6 nm or greater are known not to be able to pass through this filtration membrane (Bujie Du et al, "Transport and interactions of nanoparticles in the kidneys", Nat. Rev. Mater., 3 (2018), pp. 358-374).

The size of the particle that allows renal excretion described in the above literature is in the case of human kidneys. On the assumption of a human, the upper limit of the average particle size of the metal nanoparticle is preferably less than 6 nm and may be 5.5 nm or less, 5.25 nm or less, 5 nm or less, 4.75 nm or less, 4.5 nm or less, 4.25 nm or less, or 4 nm or less. The size of the particles that can pass through the glomerular filtration membrane of an animal does not vary greatly between different types of animals. Thus, the upper limit of the average particle size of the metal nanoparticle may be the same size as that described above regardless of the type of animal or may be slightly larger or smaller than the above size depending on the type of animal. In other words, the upper limit of the metal nanoparticle can be said to be the size that allows excretion by the kidneys of an imaging target animal.

In the present specification, the "average particle size" of the metal nanoparticle means an average value of particle diameters of many particles of the metal nanoparticle obtained by analyzing an image captured by an electron microscope using an ImageJ/Fiji.

The element for forming the metal nanoparticle is any element that can absorb X-rays and is not particularly limited, but the metal nanoparticle is preferably composed of an element with high X-ray absorption performance. In general, an element with a larger atomic number tends to have higher X-ray absorption performance. Thus, the metal nanoparticle is preferably composed of an element with a larger atomic number. Specific examples include elements belonging to the fourth or higher period of the periodic table. An element in the fifth period or higher of the periodic table, that is, an element with an atomic number of about 40 or higher is suitably used as an element constituting the metal nanoparticle because such an element has high X-ray absorption performance and can practically perform X-ray imaging. More specific examples of such an element include Ag, I, Ba, Au, Bi, Gd, Pt, Ru, Rh, Pd, Os, and Ir. Among these, gold (Au), platinum (Pt), or an alloy of gold and platinum is particularly preferred in view of an X-ray attenuation coefficient of an atom.

The metal nanoparticle is to be produced by a known method and can be produced, for example, by reducing a solution containing a metal element. For a gold nanoparticle, a specific synthesis procedure is shown in Examples described later. In addition, a platinum nanoparticle is to be produced with reference, for example, to William W. Bryan et al., "Preparation of THPC-generated silver, platinum, and palladium nanoparticles and their use in the synthesis of Ag, Pt, Pd, and Pt/Ag nanoshells", RSC Adv., 2016, 6, 68150-68159, and Hideyuki Nagao et al., "Synthesis of Platinum Nanoparticles by Reductive Crystallization Using Polyethyleneimine", Chem. Eng. Technol. 2017, 40, No. 7, 1242-1246. Furthermore, an alloy of gold and platinum is to be produced with reference, for example, to Yi Cao et al., "Fe(II)-Assisted one-pot synthesis of ultra-small core-shell Au-Pt nanoparticles as superior catalysts towards the HER and ORR", Nanoscale, 2020, 12, 20456-20466. The size of the metal nanoparticle can be adjusted by the concentration of the raw material and the reaction time.

For fine particles prepared with a metal, sub-nanoclusters are known. Sub-nanoclusters are sub-nano-order fine particles with a size of 1 nm or less composed of a relatively small number of atoms. However, sub-nanoclusters exhibit molecular-like optical transitions in absorption and emission and are greatly different in both structure and physical properties from metal nanoparticles prepared with the same element. Thus, the metal nanoparticle disclosed in the present application and sub-nanoclusters are completely different entities. In addition, sub-nanoclusters are used for applications, such as a catalyst, different from the application for X-ray contrast imaging disclosed in the present application.

### Protein

The protein is used to delay the renal excretion time of the metal nanoparticle by forming the complex together with the metal nanoparticle with a size that would otherwise allow rapid renal excretion.

The protein is not particularly limited as long as it can delay the renal excretion time of the metal nanoparticle but is preferably selected from functional proteins. Proteins can be broadly classified into two types: (1) structural proteins constituting organisms; such as collagen constituting bones, cartilages, tendons, and the like; and keratin constituting hairs, nails, and the like; and (2) functional proteins involved in chemical reactions in the body, such as transporting enzymes and substances into the body. The complex disclosed in the present application needs to be excreted by the kidneys after a predetermined time has elapsed. Thus, the protein used in the complex is preferably a functional protein, which is more easily degraded than a structural protein.

Known examples of functional proteins include enzymes, which regulate catalysis and metabolism; hormones and cytokines, which carry information; transport proteins and storage proteins, which are involved in transport and storage of substances; and antibodies, which constitute an immune mechanism. Among these, a transport protein contained in the blood is a component contained in the blood in a normal state and thus is less likely to cause rejection when a complex containing the component is administered into a living body. Thus, from the viewpoint of biocompatibility, a protein contained in the blood is preferred. Many of the proteins contained in the blood are transport proteins. Thus, a transport protein may be said to be preferred. In addition, as shown in Examples described later, for the complex disclosed in the present application, the protein is degraded in the blood, and the metal nanoparticle is excreted by the kidneys accordingly. Thus, a protein degradable in the blood can be said to be preferably used as the protein. Examples of the preferred protein include albumin, globulin, transferrin, ceruloblasmin, and lactoferrin. Furthermore, examples of the preferred protein other than proteins contained in the blood and transport proteins include avidin and streptavidin.

The types of proteins described above are merely specific examples of the protein, and the protein is not limited to the exemplified proteins. The protein may be another protein as long as it achieves the functions required for the complex disclosed in the present application. In addition, specific examples of the protein also include proteins in which a partial sequence of the proteins exemplified above is substituted or deleted to the extent that the functions required for the complex disclosed in the present application are achieved. Furthermore, the proteins may be used in combination, as necessary.

The complex according to an embodiment is not particularly limited as long as it contains the metal nanoparticle and the protein. For example, cysteine, an amino acid constituting proteins, contains a thiol group (-SH). It is also known that a metal binds to a S atom. Thus, a complex of a metal nanoparticle and a protein can be formed by removing an H atom from the thiol group of cysteine and binding a S atom to a metal.

As described above, the complex can be formed by directly binding the metal nanoparticle and the protein, but the metal nanoparticle and the protein may optionally and additionally be linked via a linker. For example, the metal nanoparticle and the protein are linked via a linker using a linker compound having a thiol group and a carboxyl or amino group.

In the case of using a linker compound containing a thiol group (-SH) and a carboxyl group (-COOH), the S atom of the thiol group binds to the metal nanoparticle (coordinate bond), and the carboxyl group binds to an amino group (-NH₂) of the protein via an amide bond. As a result, the metal nanoparticle and the protein are linked via a linker, and the complex can be formed accordingly.

In the case of using a linker compound containing a thiol group (-SH) and an amino group (-NH₂), the S atom of the thiol group binds to the metal nanoparticle (coordinate bond), and the amino group binds to a carboxyl group of the protein via an amide bond. As a result, the metal nanoparticle and the protein are linked via a linker, and the complex can be formed accordingly.

The linker compound is not particularly limited as long as it contains a thiol group and a carboxyl or amino group and can bind to the metal nanoparticle and the protein. Specific examples of the linker compound containing a thiol group and a carboxyl group include glutathione (reduced) represented by Formula (1) below, mercaptopropionic acid represented by Formula (2) below, and poly(ethylene glycol) 2-mercaptoethyl ether acetic acid represented by Formula (3) below.

The poly(ethylene glycol) 2-mercaptoethyl ether acetic acid represented by Formula (3) preferably has a molecular weight of approximately 500 to 7500, and n in Formula (3) is to be appropriately adjusted to obtain the above molecular weight. Specific examples of the compound represented by Formula (3) include HS-PEG3500-COOH and HS-PEG7500-COOH available from Sigma-Aldrich.

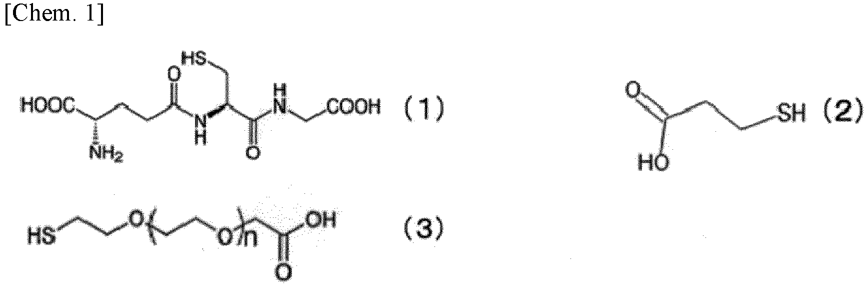

Specific examples of the linker compound containing a thiol group and an amino group include 2-aminoethanethiol represented by Formula (4) below, HS-PEG-NH₂ (M.W. of 400 to 5000, available from NANOCS, etc.) represented by Formula (5), and HS-(CH2)₁₁-NH₂ (hydrochloride) represented by Formula (6).

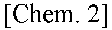

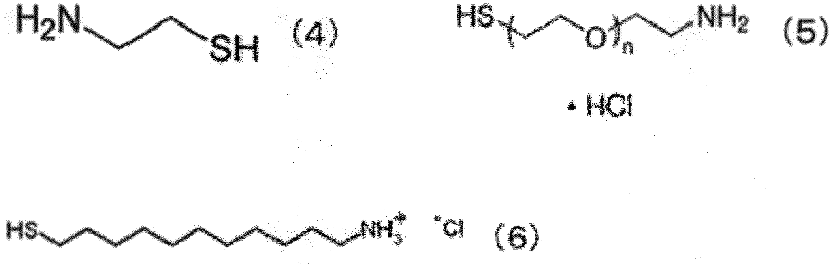

The complex disclosed in the present application contains the metal nanoparticle and the protein, and this can adjust the residence time in the blood. Thus, the complex disclosed in the present application achieves a remarkable effect, that is, it can be used as a contrast agent for imaging structural change in angiogenesis of cancer tissues over time, but its use is not limited to angiography. Applications other than angiography are not limited as long as the imaging target is a tissue that can be imaged by X-ray CT. For example, the complex may be used as a contrast agent for imaging a lymphatic vessel. In other words, the complex disclosed in the present application can be used as a contrast agent for a vessel (blood vessel and lymphatic vessel).

The size of the complex is to be appropriately adjusted according to the size of a target blood vessel, such as an artery, a vein, or a capillary vessel; the size of a lymphatic vessel; and an imaging target, such as a human or an experimental mouse. Among components contained in human blood, for example, red blood cells have a substantially disk shape with a diameter of about 7.5 µm, and lymphocytes have a diameter of about 10 to 15 µm. Thus, for example, adjusting the size of the complex to 150 nm or less eliminates a risk of clogging a blood vessel and a lymphatic vessel. The size of the complex can be adjusted by changing the amounts of the linker-bound metal nanoparticle and the protein in a method for producing the complex described later.

The complex disclosed in the present application is preferably retained in the blood until completion of X-ray CT imaging after administration and is preferably excreted by the kidneys by the time of the next X-ray CT imaging. Thus, a half-life in blood of the complex is to be adjusted to approximately 0.5 hours to 12 hours, preferably approximately 0.75 hours to 6 hours, and more preferably approximately 1 hour to 3 hours. Adjusting the half-life in blood to the time described above enables visualization particularly of structural change in angiogenesis of cancer tissues over time. As a matter of course, the half-life in blood may be shorter or longer than the above half-life in blood in the case of imaging a temporary structure instead of imaging structural change over time or using a typical X-ray CT imaging device. The half-life in blood can be adjusted by changing the type of protein or the size of the complex. The "half-life in blood" means the time it takes for the X-ray CT value of the complex to decrease by half from the maximum value after administration in vivo. However, the shortest time at which X-ray CT imaging can be started after administration of a contrast agent is about 5 minutes, and thus the measurement value at the time when X-ray CT imaging is started 5 minutes after administration of the contrast agent is defined as the "maximum value after administration in vivo" in the present specification.

### Embodiments of Vascular Contrast Agent and X-ray Contrast Agent

Using the complex disclosed in the present application as a vascular contrast agent enables visualization, for example, of structural change in blood vessels over time. However, the applications of the complex are not limited to those for vascular imaging. The complex contains the metal nanoparticle that absorbs X-rays and thus can also be used as an X-ray contrast agent for objects other than vessels.

For the X-ray contrast agent, X-ray absorption is exponentially proportional to the atomic number, and a higher contrast is obtained as the amount of metal nanoparticle increases (as the size of the metal nanoparticle increases when the number of particles is the same). On the other hand, for contrast agents, magnetic resonance imaging (MRI) contrast agents are also known. However, in imaging by MRI, the amount of a contrast agent does not always correlate with the contrast. Thus, MRI contrast agents and X-ray CT contrast agents are different technologies with different development directions.

When the complex is used as a vascular contrast agent and/or an X-ray contrast agent, the complex is to be dispersed in a medium: such as water; physiological saline; a buffer, such as a Tris-HCl buffer, a phosphate buffer, or a citrate buffer. In addition, a salt, such as sodium chloride; a sugar, such as mannitol, glucose, sucrose, or sorbitol may be added, as necessary. Adding the salt and/or sugar can make the contrast agent isotonic to the osmotic pressure in the body.

### Embodiment of Method for Producing Complex

The complexes can be produced using the metal nanoparticle, protein, and linker compound described above. A method for producing the complex includes:
(1) preparing a linker-bound metal nanoparticle by reacting the metal nanoparticle with the linker compound to bind a S atom of the thiol group to the metal nanoparticle; and
(2) binding the carboxyl group or the amino group of the linker-bound metal nanoparticle to an amino group or a carboxyl group of the protein via an amide bond by reacting the linker-bound metal nanoparticle with the protein.

In the present specification, the "linker-bound metal nanoparticle" means a reaction product of the linker compound and the metal nanoparticle as is evident from the above reaction. In the linker-bound metal nanoparticle, the thiol group of the linker compound is bound to the metal nanoparticle, but the carboxyl group or the amino group of the linker compound is present in an unreacted state.

In the method for producing the complex, first, the linker compound is bound to the metal nanoparticle. Although the metal nanoparticle has high aggregation properties, binding the linker compound to the metal nanoparticle first makes the metal nanoparticle less likely to aggregate. That is, the linker compound functions as a dispersion stabilizer (aggregation inhibitor) of the metal nanoparticle in producing the complex, in addition to the function of binding the metal nanoparticles and the protein. The metal nanoparticle and the thiol group are to be bound (coordinate bond) by a known method.

A step of binding the linker compound to the protein is not particularly limited as long as a carboxyl group and an amino group can be covalently bound. For example, an amide bond is to be utilized, which allows the use of a simple, convenient, and efficient coupling agent (cross-linker). As shown in Examples described later, the residence time of the complex (contrast agent) in the blood can be adjusted by adjusting the amount of the cross-linker when the carboxyl group or the amino group of the linker compound is bound via an amide bond to an amide group or a carboxyl group of a side chain of the protein. Thus, before performing the step of binding the linker compound and the protein, a step of calculating the amount of the cross-linker to be added may be performed to prepare a complex with a desired residence time in the blood. The amount of the cross-linker to be added is to be calculated by conducting an experiment in advance on the correlation between a plurality of types of complexes with different added amounts of the cross-linker and the residence time in the blood. Examples of the cross-linker include a combination of water-soluble carbodiimide (WSC, which may be referred to as EDC) used as a condensing agent and N-hydroxysuccinimide (NHS) used as a carboxylic acid activator. For NHS, sulfo-NHS, a water-soluble analog of NHS, may be used. When the amount of the cross-linker to be added is changed, the addition amount may be changed while the ratio of WSC and NHS is kept the same, or the ratio of WSC and NHS may be changed.

As described above, in the method for producing a complex disclosed in the present application, a step of preparing a linker-bound metal nanoparticle by binding a metal nanoparticle and a linker compound is first performed, and then a step of binding the linker-bound metal nanoparticle and a protein is performed. On the other hand, in Patent Document 2, a step of preparing "Tf-PEG-SH" is first performed, and then a step of binding "Tf-PEG-SH" and a metal fine particle is performed. Comparison of the method for producing a complex disclosed in the present application with the method for producing a complex described in Patent Document 2 reveals the following differences due to the different order of the production steps.

### (1) Difference in Structure of Produced Complex

In the complex disclosed in the present application, the protein functions as a core (center) and the metal nanoparticle is formed on the surface of the protein core. On the other hand, in Patent Document 2, the metal fine particle functions as a core, and "Tf-PEG-SH" is formed on the surface of the metal fine particle. Thus, for the complex disclosed in the present application, the particle size of the complex can be adjusted by adjusting the condensation of the protein without changing the metal nanoparticle size. On the other hand, the particle size of the complex described in Patent Document 2 depends on the particle size of the metal fine particle. Thus, for the complex described in Patent Document 2, the particle size of the complex can be adjusted by increasing the particle size of the metal fine particle. However, as described above, with an excessively increased particle size of the metal fine particle, the renal excretion ability would be lost.

### (2) Improvement of Convenience of Manufacturing Process

"Tf-PEG-SH" of Patent Document 2 can form a dimer "Tf-PEG-S-S-PEG-Tf' by an oxidation-reduction reaction. Thus, attention need be paid to the stability of "Tf-PEG-SH" in the production process. On the other hand, in the production method disclosed in the present application, the linker-bound metal nanoparticle is not dimerized after the linker compound binds to the metal nanoparticle. Thus, this improves the convenience of the manufacturing process.

### Embodiment of Imaging Method for Capturing Structural Change in Vessel

When the complex disclosed in the present application is used, structural change in a vessel can be imaged. An imaging method for capturing structural change in a vessel includes administering the complex to an animal and performing X-ray CT imaging, and after an appropriate time has elapsed, administering a new complex and performing X-ray CT imaging (the imaging step).

The imaging target animal is not particularly limited as long as it has a vessel. Examples include humans, mice, rats, rabbits, pigs, and monkeys. Humans may be excluded from the animals.

The complex is to be administered to an animal in the same manner as administration of a common vascular contrast agent. For example, the complex may be administered by injection or may be continuously administered by an injector or the like.

The imaging step is not particularly limited as long as structural change in a vessel can be captured by administering the complex and performing X-ray CT imaging, and after an appropriate time has elapsed, administering a new complex and performing X-ray CT imaging. The appropriate time is to be appropriately selected according to the imaging target and the imaging purpose. In the present specification, "structural change in a vessel" means change in shape of a blood vessel or a lymphatic vessel over time, such as, for example, angiogenesis in a tumor or the like or change in shape of an existing blood vessel over time. The structural change in a vessel can be captured by performing the imaging step twice or more. On the other hand, the upper limit of the number of times of the imaging step is not particularly specified and is to be any number of times that enables the observation of structural change in an imaging target vessel. In addition, the interval of the X-ray CT imaging is not particularly limited as long as change in the structure of a vessel can be visualized, and the interval is to be appropriately adjusted according to structural change in an imaging target vessel. For example, for imaging angiogenesis, imaging is to be performed every 12 hours to 48 hours, preferably every 18 hours to 36 hours, and more preferably every 24 hours. As a matter of course, the above time is merely an example, and the interval between the imaging steps may be shorter or longer than the above time. Visualization of a lymphatic vessel is also important in elucidating the mechanism of cancer metastasis and visualization of a sentinel lymph node of human cancer. Also for imaging a lymphatic vessel, the time is to be appropriately adjusted according to structural change in an imaging target lymphatic vessel as in imaging a blood vessel. In addition, as described above, for the contrast agent disclosed in the present application, the residence time in the blood can be adjusted. Thus, before administration of the complex, a step of selecting (or a step of preparing) a contrast agent with a suitable residence time in the blood may be performed according to the interval of performing X-ray CT imaging.

The embodiments disclosed in the present application will be specifically described by illustrating examples below; however, these examples are merely for describing the embodiments. The embodiments do not limit or do not express limitation of the scope of the invention disclosed in the present application.

### Examples

### Preparation 1 of Complex and Contrast Agent

### Example 1

A complex was prepared by the procedure described below.

### (1) Synthesis of Gold Nanoparticle

To 235.0 mL of a 0.001 M HAuCl₄ aqueous solution at 25°C, 7.5 mL of a 0.07 M tetrakis(hydroxymethyl)phosphonium chloride (THPC) aqueous solution adjusted just before use was added, and then 7.5 mL of a 1.0 M NaOH aqueous solution was added. The mixture was stirred for 15 min, and a gold nanoparticle (sAuNP) was prepared. The color of the solution changed to brown after 15 min, suggesting the sAuNP formation. HAuCl₄ available from FUJIFII,M Wako Pure Chemical Corporation, 99%, was used, and a reducing agent, THPC, available from Tokyo Chemical Industry (approximately 80% in water) was used.

### (2) Binding of Gold Nanoparticle And Linker Compound

To modify (bind) a linker compound, glutathione (GSH; FUJIFELM Wako Pure Chemical Corporation), to the sAuNP surface, 2 mL of 0.25 M GSH aqueous solution was added to a sAuNP colloidal solution. The mixture was reacted for 12 h, and an sAu/GSH colloidal solution was prepared. After the reaction, the solvent was removed from the sAu/GSH colloidal solution by an evaporator, and the colloidal solution was concentrated to a total amount of about 1/10. The concentrated sAu/GSH colloidal solution was washed by dialysis in 2 L of ultrapure water for 24 h using a 12-14 kDa dialysis tube (Visking tube, AS ONE). The washed sAu/GSH colloidal solution was powdered by removing the dispersion medium using an evaporator again. The powdered sAu/GSH was then redispersed in phosphate-buffered saline (PBS). The estimated final Au concentration was 0.5 M on the assumption that all HAuCl₄ was reduced in the above process and there was no loss in the washing and concentration processes. The redispersed solution exhibited an orange color, and no precipitate or the like was observed.

The sAu/GSH can also be prepared by mixing a HAuCl₄ aqueous solution and a glutathione aqueous solution and stirring without using THPC as a reducing agent, but the reaction takes about two weeks. On the other hand, THPC was used as a reducing agent in the production method disclosed in the example, and thus this enabled the sAu/GSH to be prepared in about half a day. Using THPC as a reducing agent greatly increases the synthesis efficiency.

### (3) Binding of Linker-Bound Gold Nanoparticle and Protein

To 1 mL of the concentrated sAu/GSH colloidal solution, 100 mg of 1-ethyl-3-(3-dimethylaminopropyl) carbodiimide hydrochloride (EDC; DOJINDO, 98%) and 100 mg of sulfo N-hydroxysulfosuccinimide sodium salt (sulfo-NHS; Tokyo Chemical Industry, 98%) were added, and the mixture was stirred at 37°C for 30 min. Then, 1 mL of an 80 mg/mL lactoferrin (LF, MW: 80 kDa, FUJIFILM Wako Pure Chemical Corporation, 95%) PBS solution was added, and the mixture was reacted at 37°C for 12 h. After the reaction, a sAu/GSH-LF colloidal solution (complex) was prepared by washing and concentrating in the same manner as for the sAu/GSH. The solution exhibited a dark orange color, and no aggregate or precipitate was observed. Thus, colloidal stability was found high also after the sAu/GSH and LF were covalently bound (via an amide bond) to form a complex. The prepared colloidal solution of the complex was used as it was as a contrast agent.

### Comparative Example 1

The colloidal solution of the sAu/GSH not subjected to "(3) binding of linker-bound gold nanoparticle and protein" in Example 1 was used as it was as a contrast agent of Comparative Example 1.

### Comparative Example 2

In 233 mL of ultrapure water, 99.4 mg of tetrachloroauric (III) acid tetrahydrate (HAuCl₄·4H₂O) was dissolved, and the mixture was heated and boiled. While the chloroauric acid aqueous solution was continuously stirred, 28 mL of 39 mM sodium citrate (FUJIFILM Wako Pure Chemical Corporation, 99%) aqueous solution was added, and the mixture was stirred at a constant rotation speed for 30 min. After the solution was cooled to 25°C, to modify the gold nanoparticle surface with polyethylene glycol (PEG), 99.4 mg of thiol carboxylic PEG (HOOC-PEG-SH, MW: 5000, Nanocos Inc.) was added to the gold nanoparticle colloidal solution. The mixture was stirred at 25°C for 8 h, and a AuNP-PEG was prepared. The prepared AuNP-PEG colloidal solution was centrifugally washed under conditions of 15000 rpm and 45 min. Thereafter, centrifugal concentration was repeated several times under conditions of 15000 rpm and 60 min, and a AuNP-PEG colloidal solution with a final Au concentration of 0.5 M was produced. PEG is a dispersion stabilizer for the gold nanoparticle. The prepared AuNP-PEG colloidal solution was used as it was as a contrast agent of Comparative Example 2.

### Comparative Example 3

Iopamidol (Iopamiron injection 300 available from Bayer Yakuhin, Ltd.), a commercially available iodine-based angiographic contrast agent, was used as Comparative Example 3.

### Evaluation Method 1 of Prepared Nanoparticles

The morphologies of the nanoparticles were evaluated using a transmission electron microscope (TEM) (JEM-2100, JEOL) under a condition of 200 kV. Samples for TEM observation were prepared by dropping the colloidal solution onto a collodion-coated copper grid (JEOL) and evaporating the solvent. The volume average particle sizes were calculated by measuring many particle diameters using an ImageJ/Fiji. Ultraviolet-visible (UV-Vis) spectra of the particulate colloidal solutions were measured using an Ultrospec 700 spectrophotometer (GE Healthcare Life Sciences). Agarose gel electrophoresis was performed using a 1% agarose (Promega) gel in 50% tris-acetate-EDTA buffer (TAE; Nippon Gene) (operating conditions: 100 KV, 10 min).

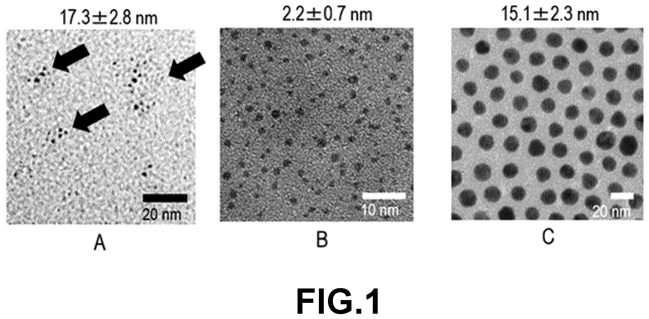

A TEM photograph of the sAu/GSH-LF prepared in Example 1 is shown in FIG. 1A, a TEM photograph of the sAu/GSH prepared in Comparative Example 1 in FIG. 1B, and a TEM photograph of the AuNP-PEG prepared in Comparative Example 2 in FIG. 1C. The average particle size of the sAu/GSH-LF prepared in Example 1 was 17.3 ± 2.8 nm. In Example 1, the complex is formed by the gold nanoparticle together with the protein and thus is not spherically shaped. Thus, the average particle size was calculated based on the length in the long axis direction of the complex portion where particles of the gold nanoparticle gathered as indicated by the arrows in FIG. 1A. The sAu/GSH of Comparative Example 1 was determined to have a monodispersed substantially spherical shape with an average particle size of 2.2 ± 0.7 nm. The AuNP-PEG of Comparative Example 2 was also determined to have a monodispersed substantially spherical shape with an average particle size of 15.1 ± nm.

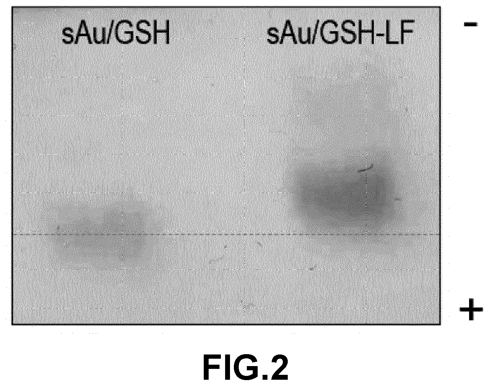

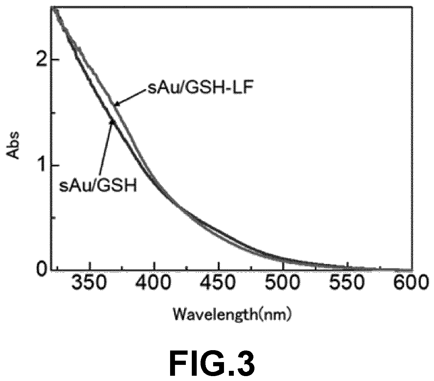

A photograph after electrophoreses of the sAu/GSH-LF colloidal solution prepared in Example 1 and the sAu/GSH colloidal solution prepared in Comparative Example 1 is shown in FIG. 2. UV-VIS spectral measurement results of the sAu/GSH-LF colloidal solution prepared in Example 1 and the sAu/GSH colloidal solution prepared in Comparative Example 1 are shown in FIG. 3.

As is evident from FIG. 2, the sAu/GSH-LF did not migrate to the anode side (lower side in the photographic direction of FIG. 2) compared with the sAu/GSH. This means that the sAu/GSH-LF had an increased particle size or altered surface charge compared with the sAu/GSH. Thus, formation of the complex was confirmed by Example 1. In addition, as is evident from FIG. 3, the sAu/GSH-LF and the sAu/GSH exhibited similar extinction spectra, and a peak assigned to a surface plasmon resonance absorption (SPR) peak was not observed. Thus, this confirmed that the gold nanoparticles were not aggregated and a precipitate was not formed by the operation of binding the protein to the sAu/GSH, in which the linker compound was bound to the gold nanoparticle.

### Administration Experiment 1 of Contrast Agent to Mice

### Preparation of Model Mice

### (1) Cell Culture

BALB/c-derived colon cancer cells colon-26 (CT26: RIKEN BioResource Research Center, RCB2657) were cultured in RPMI-1640 (Life Technologies Corporation) containing 10% fetal bovine serum (FBS) under humidified conditions of 5% CO₂ and 37°C.

### (2) Preparation of Cancer-Bearing Model Mice

Male mice of 5- to 11-week-old of wild-type mice, BALB/c (Charles River Laboratories Japan), were used. To each mouse, 1 × 10⁷ colon-26 cells cultured in (1) above were subcutaneously transplanted. Animals used were handled in accordance with guidelines approved by the Institutional Animal Care and Use Committee at Tohoku University.

### Micro X-Ray CT Imaging

Imaging was performed using a micro X-ray CT device (Sky Scan 1176, Bruker) under conditions of a tube voltage of 50 kV, a tube current of 500 µA, and 35 µm/voxel, 18 µm/voxel, or 9 µm/voxel. The contrast agents synthesized in Example 1 and Comparative Examples 1 and 2 were imaged in vitro. In vivo, 200 µL of each contrast agent was injected into an anesthetized mouse from the tail vein, and CT imaging was performed once or twice. Slice images were acquired using CTAn (Bruker), and volume rendered images were acquired using CTvox. CT values were calculated by defining the value of Hounsfield Units (HU) as -1000 for air and 0 for water.

### Confirmation of Renal Excretion in Administration of Contrast Agent of Comparative Example 1 to Mouse

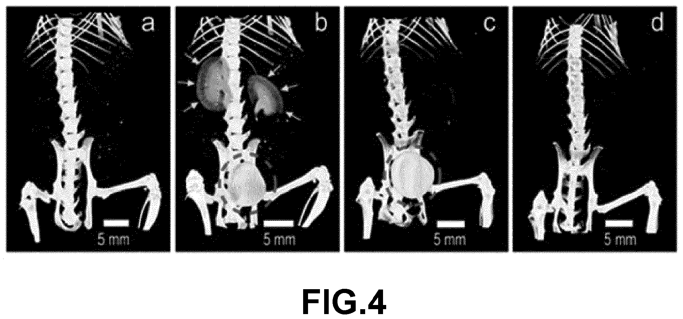

Micro X-ray CT images after administration of the contrast agent prepared in Comparative Example 1 to the tail vein of a mouse are shown in FIG. 4. FIG. 4(a) is an image before administration of the contrast agent, FIG. 4(b) is an image taken from 5 min after administration of the contrast agent, FIG. 4(c) is an image taken from 60 min after administration of the contrast agent, and FIG. 4(d) is an image taken from 24 h after administration of the contrast agent. The arrows in the figure indicate the kidneys, and the dashed line circles in the figures indicate the bladder. As shown in FIG. 4(b), the kidneys and bladder were able to be clearly visualized 5 min after administration. Furthermore, the images of the kidneys almost disappeared 60 min after administration. This confirmed that most of the contrast agent in the blood degraded, most of the gold nanoparticle accumulated in the bladder (FIG. 4(c)), and the contrast agent was excreted to the outside of the body 24 h after administration (FIG. 4(d)).

### Images of Cancer Tissue in Administration of Contrast Agent of Comparative Example 2 to Mouse

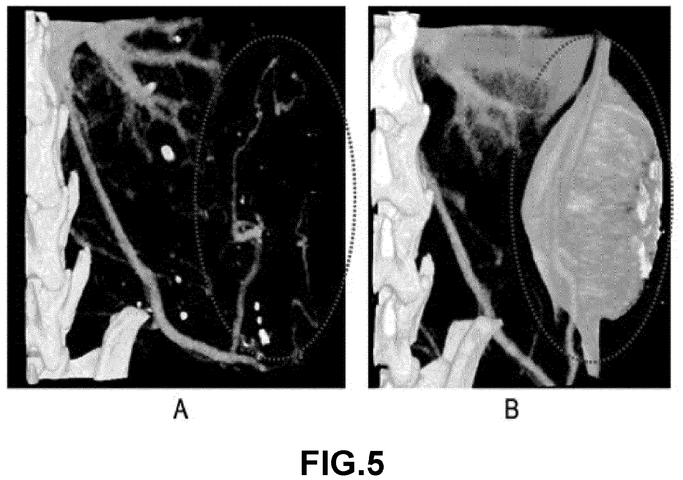

Micro X-ray CT images after administration of the contrast agent prepared in Comparative Example 2 to the tail vein of a mouse are shown in FIG. 5. FIG. 5A is an image immediately after administration of the contrast agent, and FIG. 5B is an image 3 days after administration of the contrast agent. The dashed elliptical portions in the figures are sites of tumor formation. As is evident from FIGS. 5A and 5B, these images confirmed that when the contrast agent of Comparative Example 2 was used, the contrast agent was incorporated into the stroma of the tumor tissue and retained there by the EPR effect, and thus this deteriorated the contrast of the vascular region and consequently failed to visualize structural change in the blood vessel over time at the site of tumor formation.

The results shown in FIGS. 4 and 5 revealed: (1) the gold nanoparticle with a small particle size is rapidly excreted from the kidneys; and (2) on the contrary, the gold nanoparticle with a large particle size cannot be separated in the kidneys and is retained in the stroma of the tumor tissue by the EPR effect, and this makes it difficult to visualize structural change in the blood vessel over time at the site of tumor formation.

### Measurement of Half-Life in Blood in Administration of Contrast Agents of Example 1 and Comparative Examples 1 and 3 to Mice

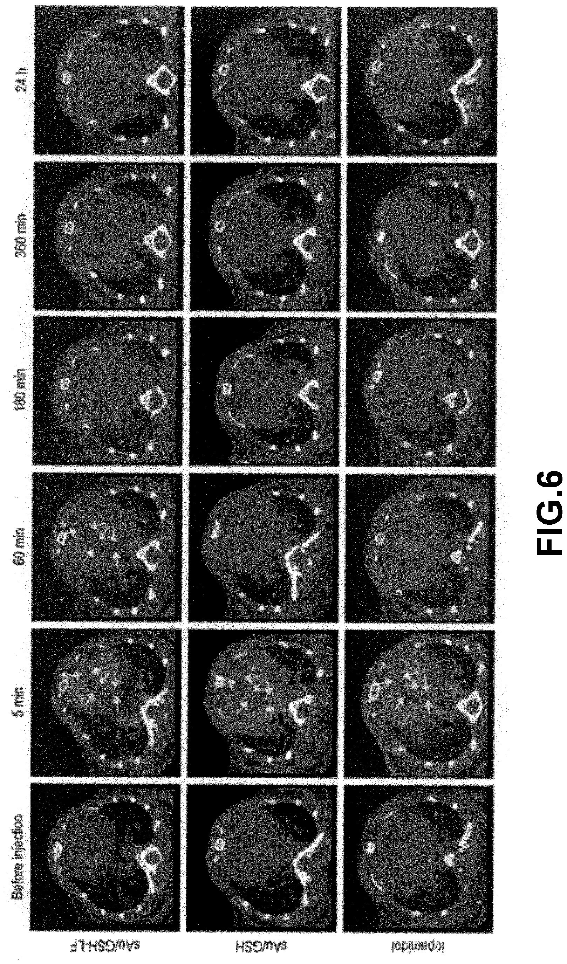

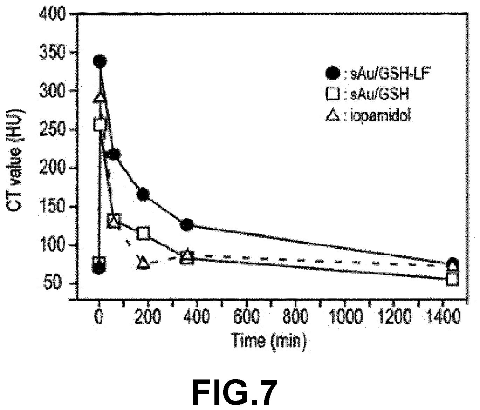

CT images of mouse hearts before and after intravenous administrations of the sAu/GSH-LF contrast agent prepared in Example 1, the sAu/GSH contrast agent prepared in Comparative Example 1, and iopamidol of Comparative Example 3 are shown in FIG. 6. In all the contrast agent administration groups, images taken from 5 min after administration revealed clear images with a level enabling distinction between the left and right ventricles. In the images taken from 60 min after administration, the left and right ventricles were distinguishable only in the mouse given the sAu/GSH-LF. Results of measuring changes in CT values of ventricles over time are shown in FIG. 7. The image before the administration of the contrast agent was taken as 0 min (before injection). The CT value in the sAu/GSH-LF administration group was 71, 337, 217, 166, 126, and 75 HU at 0, 5, 60, 180, 360 min, and 24 h, respectively. The CT value in the sAu/GSH administration group was 76, 256, 132, 115, 83, and 55 HU at 0, 5, 60, 180, 360 min, and 24 h, respectively. The CT value in the iopamidol administration group was 72, 291, 130, 75, 87, and 72 HU at 0, 5, 60, 180, 360 min, and 24 h, respectively. The half-life in blood of each contrast agent was calculated from the results shown in FIG. 7. The half-life in blood was 120 min for the sAu/GSH-LF. In contrast, the half-life in blood was less than 60 min for both the sAu/GSH and iopamidol.

The above results confirmed: (1) both the sAu/GSH and iopamidol exhibited similar behavior in blood vessels; (2) the sAu/GSH-LF prepared by binding lactoferrin to the sAu/GSH can have a longer half-life in blood than the sAu/GSH; and (3) the sAu/GSH-LF has high angiographic ability compared with the sAu/GSH and iopamidol, which have been known as angiographic contrast agents in the art.

### Images Over Time of Cancer Tissue Blood Vessels in Administration of Contrast Agents of Example 1 and Comparative Examples 1 and 2 to Mice

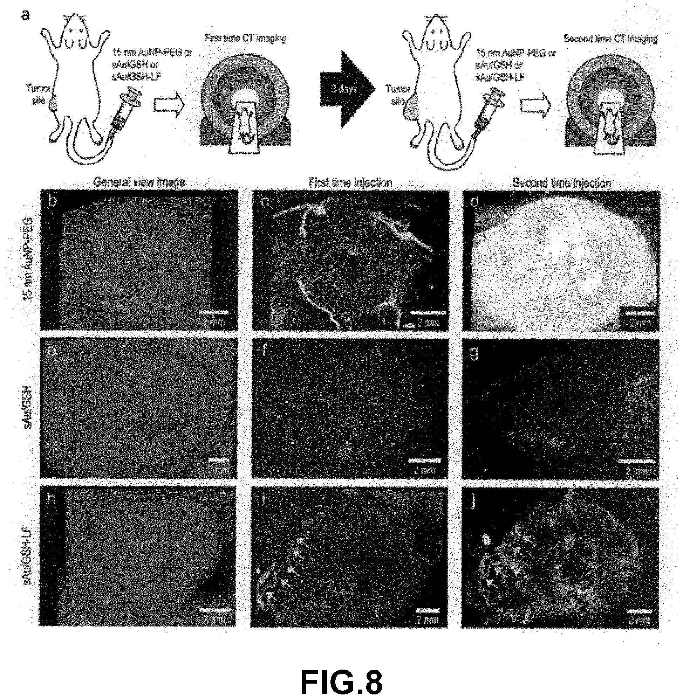

As shown in FIG. 8. the sAu/GSH-LF contrast agent prepared in Example 1, the sAu/GSH contrast agent prepared in Comparative Example 1, and the 15-nm AuNP-PEG contrast agent prepared in Comparative Example 2 were used to determine whether each contrast agent was able to visualize changes in the same blood vessels associated with tumor growth. FIG. 8a is a diagram illustrating an outline of the experiment. The contrast agent was intravenously administered to a cancer-bearing mouse, and a first X-ray CT imaging was performed after 5 min. The contrast agent was administered 3 days later (72 h) in the same manner as in the first administration, and a second X-ray CT imaging was performed after 5 min.

FIGS. 8b to 8j are CT images of tumor portions. As shown in FIGS. 8b to 8d, when the 15-nm AuNP-PEG contrast agent was administered, tumor blood vessels were clearly observed after the first administration (FIG. 8c), but in the second CT image, the entire tumor portion turned completely white, and the tumor blood vessels could not be visualized, failing to observe the structural change in the tumor blood vessels in a matter of days (FIG. 8d). This is probably because the gold nanoparticle had a size of 15 nm, thus had no excretion route from the blood and accumulated in the skin or tumor tissue.

As shown in FIGS. 8e to 8g, when the sAu/GSH contrast agent was administered, visualization of tumor blood vessels was difficult. This is probably because the sAu/GSH was excreted in urine immediately after administration of the contrast agent.

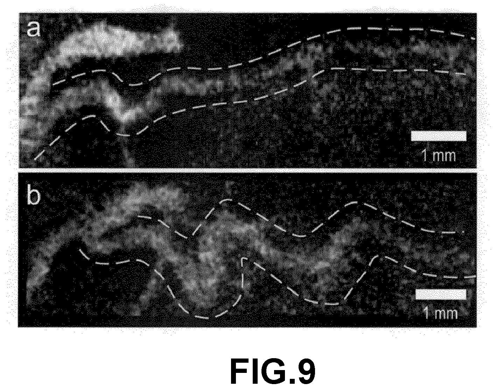

Unlike the two types of contrast agents described above, as shown in FIGS. 8h to 8j, the sAu/GSH-LF contrast agent when administered enabled visualization of tumor blood vessels in the CT image after the first administration (FIG. 8j) and enabled visualization of the same tumor blood vessels after the second administration of the contrast agent (arrows in FIGS. 8i and 8j). FIG. 9a is an enlarged image of FIG. 8i, and FIG. 9b is an enlarged image of FIG. 8j. In only 72 h, the degree of tortuosity and diameter of the same tumor blood vessel greatly changed (the vessel surrounded by dotted lines in FIGS. 9a and 9b). The degree of tortuosity (tortuosity degree) was defined and calculated as a value obtained by dividing the actual length by a straight line between two points. As a result, the tortuosity degree of the same blood vessel was determined to have changed from about 1.1 to 1.4. In addition, the maximum diameter of the blood vessel was determined to have changed from 420 µm to 515 µm. The number of tumor blood vessels that were able to be visualized in the first CT image after administration of the contrast agent was smaller for the sAu/GSH-LF (FIG. 8i) than for the AuNP-PEG (FIG. 8c). The reason is probably that the 15-nm AuNP-PEG, which was densely packed, had a high Au-atom density and thus had high imaging ability.

The above results confirmed that using as a contrast agent the complex prepared by binding the protein to the metal nanoparticle with a size that would otherwise allow renal excretion enables visualization of structural change in the same tumor blood vessel over time.

For lactoferrin, which was used as the protein in the present experiment, the half-life in blood of lactoferrin alone is known to be about 7.8 min (Y. Nojima et al., "Lactoferrin conjugated with 40-kDa branched poly(ethylene glycol) has an improved circulating half-life", Pharm. Res., 26 (2009), pp. 2125-2132). As shown in the above example, the half-life in blood of the complex is from 1 to 3 hours. Thus, the disclosure in the present application has been made based on the unexpected effect, that is, the sAu/GSH and lactoferrin when each administered alone are each rapidly excreted from the blood, but the residence time in the blood is greatly prolonged when they are used in combination.

Tumors require a lot of nutrition and oxygen to grow rapidly. To increase the supply of nutrition and oxygen to tumor tissue, cancer cells produce angiogenic factors and rapidly build tumor blood vessels. Therefore, rapidly built tumor neovessels are irregularly shaped and dilated, and thus, leakier and more fragile. In addition, the lymphatic network system is immature in tumors, and thus a material leaked from tumor blood vessels is not easily removed from the tumor site. Thus, a nanoparticle or drug with a size of about 150 nm or less permeates tumor blood vessels and are easily retained in tumor tissue. This phenomenon is called the enhanced permeability and retention (EPR) effect. When the contrast agent of Comparative Example 2 was administered, as shown in FIGS. 5 and 8, the metal nanoparticle was accumulated in tumor tissues by the EPR effect, failing to visualize structural change in the blood vessel over time.

On the other hand, the sAu/GSH-LF, the complex of Example 1, had a size of 17.3 ± 2.8 nm, which was almost the same size as 15 nm of the AuNP-PEG of Comparative Example 2. However, the sAu/GSH-LF had a small amount of Au and thus is less likely to be visualized even if a similar EPR effect occurred, and the sAu/GSH-LF was degraded, and thus almost no influence of the EPR effect was observed. This is evident from the image shown in FIG. 8j, in which the entire tumor tissue is not imaged unlike the image in FIG. 8d. Thus, the complex disclosed in the present application has the functions of (1) being almost unsusceptible to the EPR effect despite its size not subject to renal excretion and (2) having a half-life in blood sufficient for imaging the blood vessel of the tumor tissue, and thus achieves a remarkable effect of enabling visualization of structural change in blood vessels over time.

### Preparation (2) of Complex and Contrast Agent

### Example 2

A complex (a colloidal solution obtained by forming a complex of the sAu/GSH and human serum albumin) was prepared by the same procedure as in Example 1 except that 1 mL of a 50 mg/mL albumin (FUJIFILM Wako Pure Chemical Corporation) PBS solution was added instead of the lactoferrin PBS solution of Example 1. The complex prepared in Example 2 (which may also be described as the colloidal solution or the contrast agent) may be described as "sAu/GSH-hSA 1".

### Example 3

A complex (a colloidal solution obtained by forming a complex of the sAu/GSH and human serum albumin) was prepared by the same procedure as in Example 2 except that the amount of 1-ethyl-3-(3-dimethylaminopropyl) carbodiimide hydrochloride (EDC; DOJINDO, 98%) added was 400 mg and the amount of sulfo N-hydroxysulfosuccinimide sodium salt (sulfo-NHS; Tokyo Chemical Industry, 98%) added was 400 mg. The complex prepared in Example 3 (which may also be described as the colloidal solution or the contrast agent) may be described as "sAu/GSH-hSA 2".

### Evaluation Method 2 of Prepared Nanoparticles

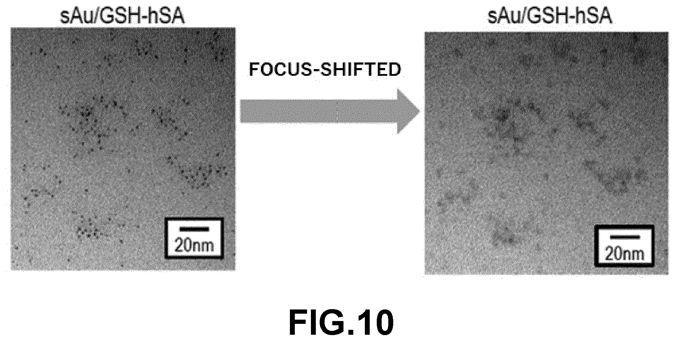

The complexes prepared in Examples 2 and 3 were evaluated by the same procedure as in "evaluation method 1 of prepared nanoparticles" above (however, the time of electrophoresis was changed to 20 min). FIG. 10 shows TEM photographs of sAu/GSH-hSA 1 prepared in Example 2. TEM photographs of sAu/GSH-hSA 2 prepared in Example 3 are omitted. The left side of FIG. 10 is a photograph focusing on the produced gold nanoparticle, and the right side is a photograph in which the focus was shifted from the focus plane of the gold nanoparticle. As is evident from the photograph on the left side of FIG. 10, in sAu/GSH-hSA 1 prepared in Example 2, particles of the gold nanoparticle aggregated as in Example 1 (FIG. 1A). In addition, a low-contrast material was also observed around the gold nanoparticle in the right photograph after the focus shift. This confirmed that the gold nanoparticle and the protein formed a complex also in Example 2.

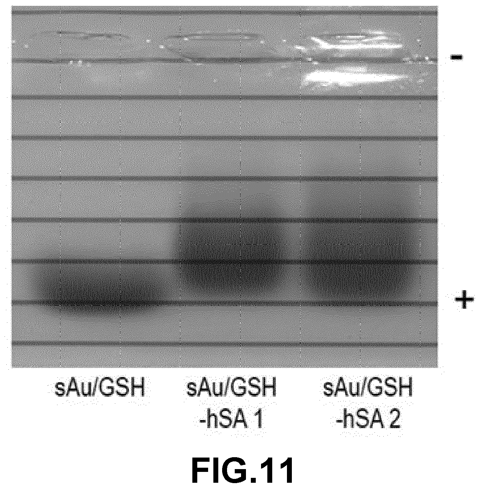

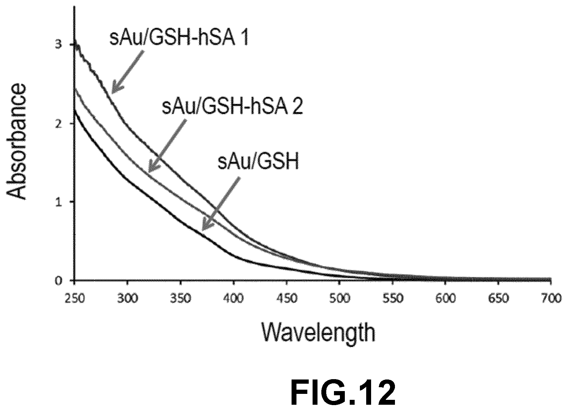

A photograph after electrophoreses of the colloidal solution (sAu/GSH-hSA 1) prepared in Example 2, the colloidal solution (sAu/GSH-hSA 2) prepared in Example 3, and the colloidal solution (sAu/GSH) prepared in Comparative Example 1 is shown in FIG. 11. UV-VIS spectral measurement results of the colloidal solutions prepared in Examples 2 and 3 and Comparative Example 1 are shown in FIG. 12.

As shown in FIG. 11, sAu/GSH-hSA 1 prepared in Example 2 and sAu/GSH-hSA 2 prepared in Example 3 did not migrate to the anode side (lower side in the photographic direction of FIG. 11) compared with the sAu/GSH of Comparative Example 1 as in Example 1. Thus, this confirmed that the complex was formed in Examples 2 and 3. In addition, as shown in FIG. 12, sAu/GSH-hSA 1, sAu/GSH-hSA 2, and the sAu/GSH exhibited similar extinction spectra, and a peak assigned to a surface plasmon resonance absorption (SPR) peak was not observed. Thus, this confirmed that in the complexes of Examples 2 and 3, the gold nanoparticle was not aggregated and a precipitate was not formed as in Example 1.

### Evaluation Method 3 of Prepared Nanoparticles

Next, the isoelectric points of sAu/GSH-hSA 1 prepared in Example 2, sAu/GSH-hSA 2 prepared in Example 3, and the sAu/GSH prepared in Comparative Example 1 were measured. For the experimental procedure, a method was used, in which the pH of the prepared sAu/GSH-hSA colloidal solution was changed in the range of 2 to 12, and zeta potential was calculated from electric mobility measured by the dynamic light scattering method. The pH of the solution was adjusted with sodium hydroxide or hydrochloric acid, and the pH value at which the zeta potential was 0 was taken as the isoelectric point.

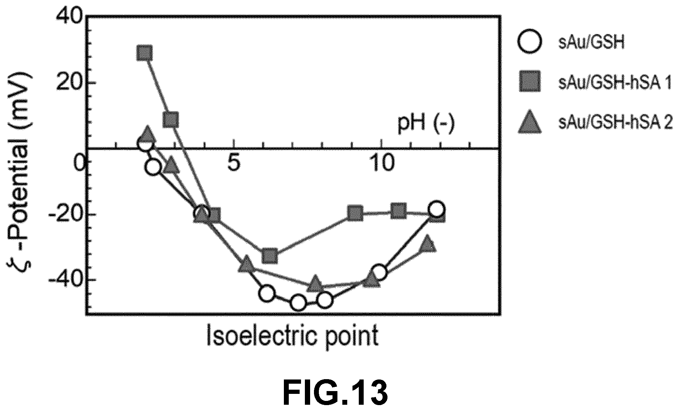

The measurement results of the isoelectric points are shown in FIG. 13. As is evident from FIG. 13, the shift of the isoelectric point was observed, suggesting that the sAu/GSH and human serum albumin formed a complex. In addition, the isoelectric point was different between Examples 2 and 3. This suggested that the structure of the complex was changed (the structures were different) by adjusting the amount of a cross-linker (a condensing agent, such as EDC, and a carboxylic acid activator, such as NHS) used in the step of binding the linker compound and the protein via an amide bond.

### Administration Experiment 2 of Contrast Agent to Mice

### Confirmation of Renal Excretion in Administration of Contrast Agents of Examples 2 and 3 and Comparative Example 1 to Mice

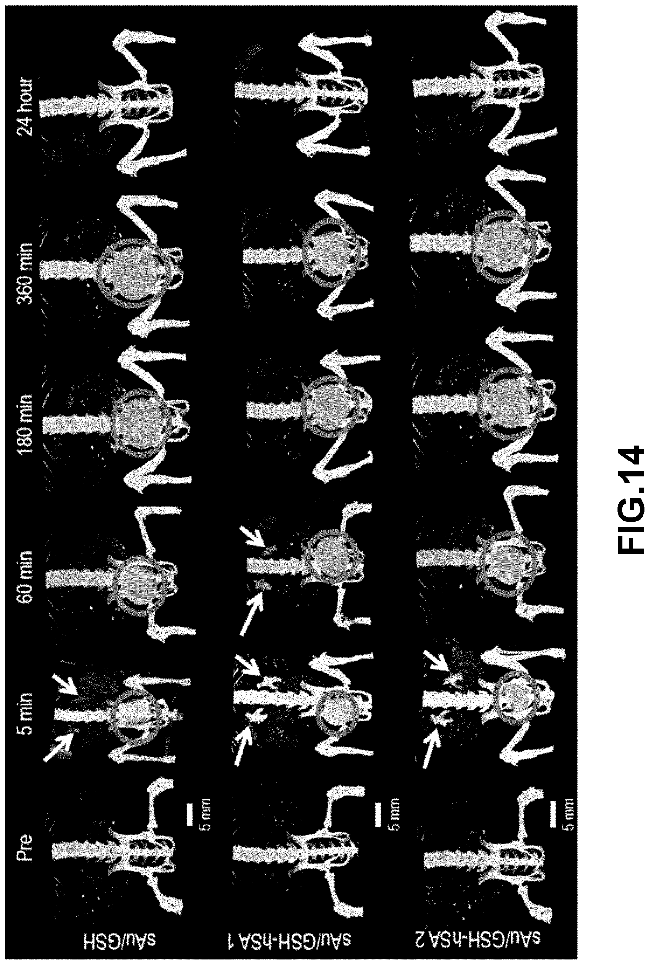

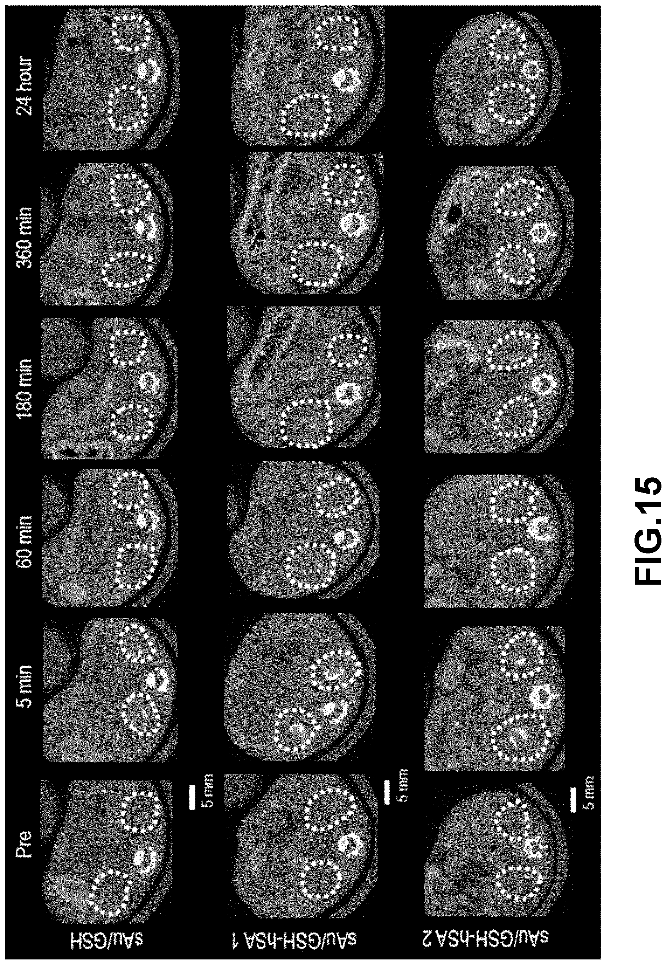

Renal excretion after administration of the contrast agent was confirmed by the same procedure as in "administration experiment 1 of contrast agent to mice" described above except that the contrast agents prepared in Examples 2 and 3 and Comparative Example 1 were used and the imaging time after administration of the contrast agent was 5 min, 60 min, 180 min, 360 min, and 24 hours. Micro X-ray CT images are shown in FIGS. 14 and 15. FIG. 14 shows CT images of mice viewed in the abdominal direction as in "administration experiment 1 of contrast agent to mice" described above. In addition, FIG. 15 shows CT images of axial cross sections of the kidneys of mice. The arrows in FIG. 14 indicate the kidneys (renal pelves), and the substantially circular lines indicate the bladders. Furthermore, the substantially circular dotted lines in FIG. 15 indicate the contours of the kidneys (renal pelves), and white contrast regions inside the dotted lines indicate the renal pelves of the kidneys.

As is evident from FIGS. 14 and 15, compared with the contrast agent of Comparative Example 1, the contrast agents of Examples 2 and 3 enabled clearer visualization of the kidneys 5 min after administration. Thus, this confirmed that the contrast agents of Examples 2 and 3 had longer residence time in the blood than the contrast agent of Comparative Example 1. Furthermore, in comparison between Examples 2 and 3, the contrast agent of Example 2 had longer residence time in the blood. This confirmed that the residence time of the contrast agent in the blood can be adjusted by changing the addition amount of the cross-linker used in the step of binding the linker compound and the protein via an amide bond.

### Measurement of Half-Life in Blood in Administration of Contrast Agents of Examples 2 and 3 and Comparative Example 1 to Mice

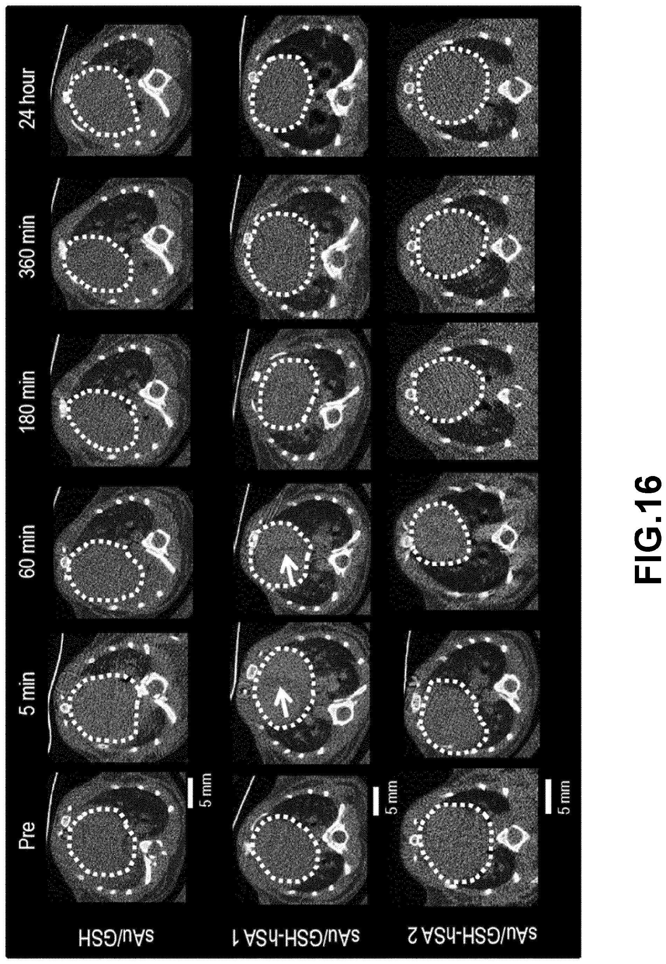

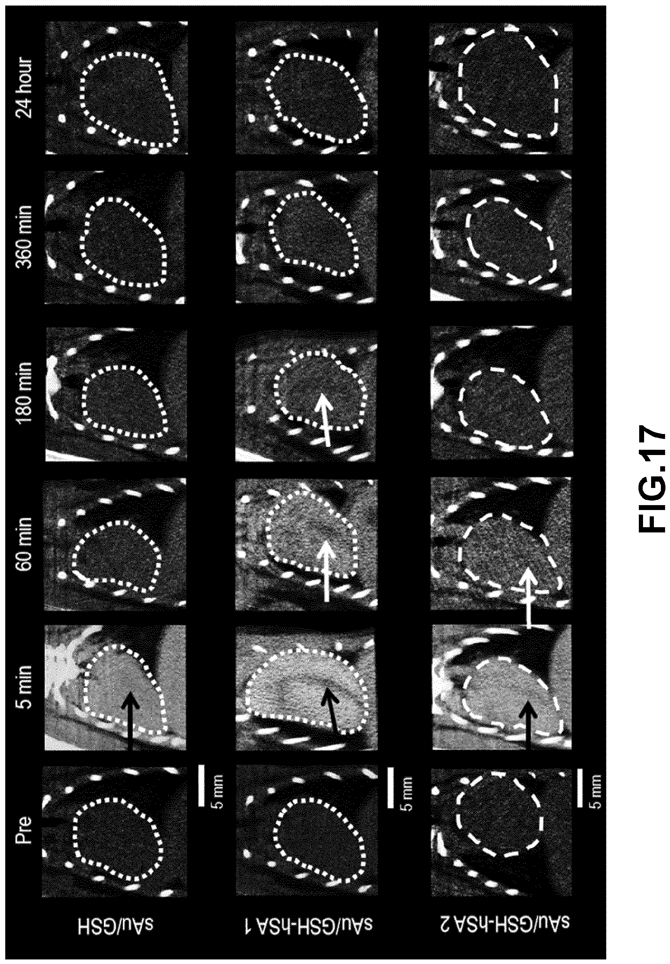

The half-life in blood of the contrast agents was measured by the same procedure as in "administration experiment 1 of contrast agent to mice" described above except that the contrast agents prepared in Examples 2 and 3 and Comparative Example 1 were used. FIGS. 16 and 17 show CT images of mouse hearts before and after intravenous administrations of the contrast agents. FIG. 16 shows CT images of axial cross sections of the hearts of mice as in "administration experiment 1 of contrast agent to mice" described above. FIG. 17 shows CT images of coronal cross sections of the hearts of mice. In addition, in FIGS. 16 and 17, the dotted line circles indicate the contour of the hearts, and the tips of the arrows (white and black) indicate the positions of the septa of the hearts.

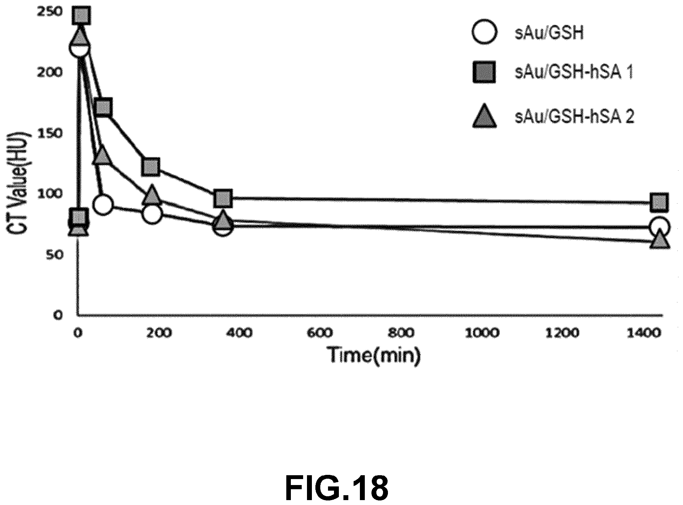

The results shown in FIGS. 16 and 17 confirmed, as in the experiment of renal excretion, that the contrast agents prepared in Examples 2 and 3 had longer residence time in the blood than the contrast agent prepared in Comparative Example 1. In addition, the results also confirmed that the contrast agent prepared in Example 2 had longer residence time in the blood than the contrast agent prepared in Example 3. FIG. 18 is a graph showing results of measuring changes in CT values of ventricles over time. The half-life in blood of each contrast agent was calculated from the results shown in FIG. 18. The half-life in blood was about 180 min for sAu/GSH-hSA 1 of Example 2 and about 100 min for sAu/GSH-hSA 2 of Example 3. In contrast, the half-life in blood was less than 60 min for the sAu/GSH of Comparative Example 1.

The above results confirmed that the residence time of the contrast agent in the blood can be adjusted by changing the addition amount of the cross-linker used in the step of binding the linker compound and the protein via an amide bond.

### Reference Example 1, Preparation of Linker-Bound Gold Nanoparticle

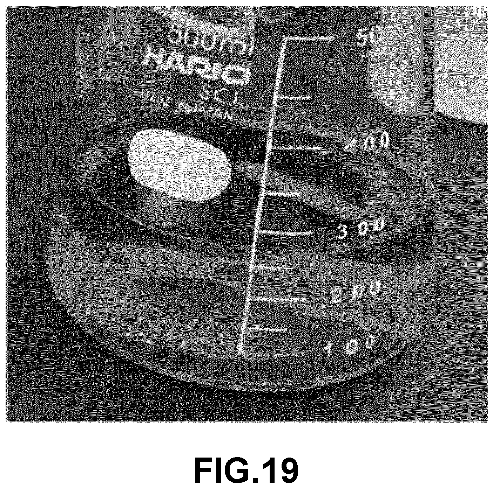

A gold nanoparticle was synthesized by the same procedure as in "(1) synthesis of gold nanoparticle" in "preparation 1 of complex and contrast agent" described above. Thereafter, a linker-bound gold nanoparticle was prepared by the same procedure as in "(2) Binding of gold nanoparticle and linker compound" described above except that 8 mL of 0.25 M 2-aminoethanethiol (available from Tokyo Chemical Industry Co., Ltd. (TCI), A0648, 2-Aminoethanethiol (>95%)) solution was added instead of glutathione, the mixture was reacted for 12 hours, and then pH was adjusted. FIG. 19 is a photograph of a 2-aminoethanethiol-bound gold nanoparticle solution prepared in Reference Example 1. As shown in FIG. 19, a clear liquid was obtained, and no precipitate or the like was observed. When glutathione is bound to the gold nanoparticle, a carboxyl group is exposed on the surface of the gold nanoparticle, whereas when 2-aminoethanethiol is bound to the gold nanoparticle, the amino group is exposed on the surface of the gold nanoparticle. Depending on the type of protein, the numbers of amino groups and carboxyl groups in the side chain vary. Thus, when the complex is prepared, the combination of the linker compound and the protein is to be designed to obtain a suitable amide bond.

### Industrial Applicability

Preparing a contrast agent with the complex disclosed in the present application enables visualization of structural change in a blood vessel over time. Thus, this is useful for the medical industry.

## Claims

1. A complex comprising a metal nanoparticle and a protein, wherein
a metal nanoparticle part in the complex has an average particle size of 1 nm or greater and 5.5 nm or less.

2. The complex according to claim 1, wherein
the metal nanoparticle and the protein are linked via a linker.

3. The complex according to claim 2, wherein
the linker before forming the complex is a compound comprising a thiol group and a carboxyl or amino group, and
in the complex,
a S atom of the thiol group is bound to a metal in the metal nanoparticle, and
the carboxyl group or the amino group is bound via an amide bond to an amino group or a carboxyl group of the protein.

4. The complex according to any one of claims 1 to 3, wherein
the protein is selected from functional proteins.

5. The complex according to any one of claims 1 to 4, wherein
the complex has a half-life in blood from 1 to 3 hours.

6. The complex according to any one of claims 1 to 5, wherein
in the complex, the protein functions as a core, and the metal nanoparticle is bound to a surface of a protein core provided by the protein.

7. The complex according to any one of claims 1 to 6, wherein
a metal in the metal nanoparticle is selected from the group consisting of gold, platinum, and alloys of gold and platinum.

8. A vascular contrast agent comprising the complex described in any one of claims 1 to 7.

9. An X-ray contrast agent comprising the complex described in any one of claims 1 to 7.

10. A method for producing a complex using a metal nanoparticle, a protein, and a linker compound, a metal nanoparticle part in the complex having an average particle size of 1 nm or greater and 5.5 nm or less, the linker compound having a thiol group and a carboxyl or amino group, and the method comprising:
preparing a linker-bound metal nanoparticle by reacting the metal nanoparticle with the linker compound to bind a S atom of the thiol group to a metal in the metal nanoparticle; and
binding the carboxyl group or the amino group of the linker-bound metal nanoparticle to an amino group or a carboxyl group of the protein via an amide bond by reacting the linker-bound metal nanoparticle with the protein.

11. An imaging method for capturing structural change in a vessel using a complex, the complex having a metal nanoparticle and a protein, a metal nanoparticle part in the complex having an average particle size of 1 nm or greater and 5.5 nm or less, and
the imaging method comprising:
administering the complex to an animal and performing X-ray CT imaging, and after an appropriate time has elapsed, administering a new complex and performing X-ray CT imaging.
